# EUROPÄISCHE PATENTANMELDUNG

(11) **EP 4 670 480 A1**
(43) Veröffentlichungstag der Anmeldung: **31.12.2025**
(21) Anmeldenummer: 25184345.4
(22) Anmeldetag: 22.06.2025
(51) Int. Cl.: A01B 77/00, A01M 17/00, A01M 21/04, A61L 2/06, B09C 1/06, F27B 7/08, F27B 7/16

(54) **VORRICHTUNG MIT RÜTTELSIEB UND BEHEIZBARER MISCHTROMMEL ZUR THERMISCHEN BEHANDLUNG VON SCHÜTTGUT**

(30) Priorität: 25.06.2024 DE 102024002068
(71) Anmelder: Richard Rupprecht GmbH, 91233 Neunkirchen a.S. (DE); Herbert Wörner Gärtnerei GmbH, 86420 Diedorf-Lettenbach (DE)
(72) Erfinder: Rupprecht, Andreas, 91233 Neunkirchen a. S. (DE); Wörner, Herbert, 86356 Neusäß (DE)
(74) Vertreter: Dreykorn-Lindner, Werner

(57) **Zusammenfassung**

Um eine Vorrichtung zur thermischen Behandlung von Schüttgut derart auszugestalten, dass diese eine kontinuierliche und energieeffiziente Beschickung ermöglicht und ein Sicherheitsrisiko für den Anwender zuverlässig vermieden wird, weist diese auf:

• ein mehrstöckiges Gestell (G) aus Rohrrahmen (RR), in welchem am unteren Tragrahmen (T1) im untersten Stock ein Trommelkörper (T) mit Mischschaufeln (M) gelagert und auf dem im Abstand darüber liegenden Tragrahmen (T2) im obersten Stock ein Rüttelsieb (A) angeordnet ist,

• eine Rutsche (RU) zur Zuführung des vorgesiebten Mischguts zu einer Trommelöffnung (E) des Trommelkörpers (T),

• einen außerhalb des Trommelkörpers (T) angeordneten Gasbrenner (B), dessen Flamme sich in das Innere des Trommelkörpers (T) erstreckt und

• ein im Innern des Trommelkörpers (T) oberhalb der Flamme angeordnetes Prallblech (PB).

Weiterhin wird diese Aufgabe durch ein Betriebsverfahren zur thermischen Behandlung von Schüttgut mittels der Vorrichtung gelöst.

## Beschreibung

Die Erfindung betrifft eine Vorrichtung mit Rüttelsieb und beheizbarer Mischtrommel zur thermischen Behandlung von Schüttgut gemäß dem Patentanspruch 1 und ein Verfahren hierzu gemäß Patentanspruch 10.

Dämpfen ist eine alternative Sterilisationsmethode für Mutterböden in der Landwirtschaft und im Erwerbsgartenbau. Das Ziel ist eine rasche und sichere Befreiung des Bodens von pflanzenschädlichen Substanzen und Organismen, wie
- Stoffwechselprodukte
- Bakterien
- Viren
- Pilze
- Nematoden
- Unkrautsamen und Wurzelunkräuter und
- andere tierische Schädlinge.

Die Flächendämpfung mit Spezialfolien (Foliendämpfung) ist eine jahrzehntelang bewährte Methode, um größere Flächen von 15 m² bis 400 m² in einem Arbeitsgang zu dämpfen. Bei richtiger Anwendung der Foliendämpfung ist diese einfach und wirtschaftlich. Hitzebeständiges und verrottungssicheres Isoliervlies spart bis zu 50 % Energie, verringert die Dämpfzeit erheblich und verbessert die Tiefenwirkung. Dämpfflächen bis 400 m² je Arbeitsgang, in 4-5 Stunden auf 25-30 cm Tiefe bei einer Temperatur von 90 °C sind durchführbar.

Eine Dämpfhaube ist eine mobile Vorrichtung, welche auf die zu dämpfende Fläche abgesetzt wird. Im Gegensatz zur Foliendämpfung entfallen die aufwandsintensiven Arbeitsschritte der Folienverlegung und -beschwerung, jedoch ist die Flächenleistung pro Arbeitsschritt abhängig von der Haubengröße entsprechend geringer. Als Nachteil ist das extreme Vernässen des Ausgangsmaterials zu sehen.

Beispielsweise ist aus der DE102010045286B4 eine Anordnung zur Sterilisation von Erdreich mit einer an ein Zugfahrzeug ankoppelbaren Dämpfvorrichtung zum Behandeln des Erdreichs mit Dampf. Um auf möglichst einfache Weise auch große Flächen und Felder zur Sterilisation behandeln zu können ist vorgesehen, dass die Dämpfvorrichtung mindestens eine mit einem Dämpfschuh verbundene Schar aufweist, dass der Dämpfschuh mindestens eine Öffnung zur Abgabe von Dampf in das Erdreich entgegen der Fahrtrichtung aufweist, und dass die Dämpfvorrichtung mit einer Mischeinrichtung zum Mischen des Erdreichs mit dem abgegebenen Dampf gekoppelt ist. Somit wird das zu behandelnde Erdreich im Anschluss an das Einbringen des Dampfs durchgemischt, so dass der heiße Dampf unter das Erdreich gehoben wird. Diese Maßnahme bewirkt, dass die Anordnung gemäß der DE102010045286B4 das zu behandelnde Erdreich besonders gründlich und effektiv sterilisiert und ermöglicht die mobile Anordnung mit einer vergleichsweise hohen Geschwindigkeit (ca. 80 bis 120 Meter in der Stunde) zu betreiben. Die Dämpfeinrichtung ist vorzugsweise als Schälpflug oder Grubber ausgebildet, besonders bevorzugt als Flügelschargrubber, wobei hohle Dämpfschuhe etwa keilförmig ausgebildet sind und eine breite, gebogene Rückseite aufweisen. Die Mischeinrichtung ist bevorzugt als Rüttelegge oder Kreiselegge ausgebildet.

Ebenfalls sind stationäre Maschinen zur Erddämpfung für Garten- und Ackerbaubetriebe seit langem bekannt. Diese weisen meist eine drehend angetriebene, etwas geneigt angeordnete und am inneren Umfang mit Vorsprüngen, Schaufeln und dergleichen versehene Trommel und eine Zerkleinerungseinrichtung für die behandelte Erde auf, wobei durch die Trommel in der Längsrichtung heiße Gase strömen, welche die bei der Drehung die Trommel mehrfach durchfallende Erde erhitzen und dämpfen. Dadurch wird diese von tierischen und pflanzlichen Schädlingen befreit, ohne dass die Erde selbst verbrennt oder ihre für Kulturzwecke wichtigen Bodenbakterien vernichtet werden. In handelsüblichen Gebinde wird die behandelte Erde vom Produktionsbetrieb dann an den Hobbygärtner verkauft.

Beispielsweise ist aus der CH348280A ein fahrbares Mehrzweckgerät, insbesondere für den gewerblichen Gartenbau mittels welchem nicht nur Erdaufbereitungsarbeiten, sondern auch andere in Gartenbaubetrieben anfallende Arbeiten, bei welchen eine Wärmequelle erforderlich ist, durchgeführt werden können. Das Mehrzweckgerät ist gekennzeichnet durch ein fahrbares Gestell, eine darauf angebrachte Heizeinrichtung, ferner zwei oder mehr darauf nach Wahl montierbare, von der erwähnten Heizeinrichtung beheizbare Einrichtungen, und zwar eine Erddämpfungstrommel, eine Lufterhitzungsanlage und/oder eine Warmwasserbereitungsanlage. Das Grundgerät besitzt einen Rahmen mit zwei Radpaaren. Der Rahmen besteht aus einem unteren Hauptrahmen aus U-Profilstahl, einem damit starr verbundenen, aus Rohren bestehenden oberen Rahmen und einem schwenkbaren Rahmen. Der Rahmen ist um eine horizontale Querachse am Hauptrahmen schwenkbar gelagert und mittels bekannter Einrichtungen, z. B. mittels eines oder mehrerer hydraulisch bewegter Kolben, in Bezug auf seine Neigung gegenüber der Horizontalen ein- und feststellbar. An dem der horizontalen Querachse gegenüberliegenden, etwas nach oben gekröpften Ende des Schwenkrahmens ist ein Ölbrenner befestigt, welcher mit seiner Haube das benachbarte Ende der auswechselbaren beheizbaren Einrichtungen teilweise umfasst. Die Einrichtung für die Erddämpfung besteht aus einer langen Trommel, welche am Innenumfang Leitbleche und an den beiden Enden Öffnungen aufweist. Durch die erste Öffnung wird die Trommel über einen mit Schüttelsieb und Ableitrinne versehenen Fülltrichter mit Erde beschickt. Der Fülltrichter ist am Rahmen ruhend befestigt, wogegen die Trommel über den Antrieb und die Reibrollen, auf welchen der Bund aufliegt, drehend angetrieben ist. Beschickt man die drehend angetriebene Trommel über das vom Motor angetriebene Schüttelsieb 46 und den Fülltrichter mit Erde, so wird diese durch die an der Innenwand der Trommel angeordneten Leitbleche bei der Drehung immer wieder angehoben und fällt, den Trommelraum durchquerend, wieder zu Boden, wobei sie allmählich, unterstützt durch die Neigung der Trommel, weiterwandert und durch die zweite (gegenüberliegende) Öffnung wieder austritt. Dabei wird die Erde von den vom Ölbrenner kommenden Flammgasen durchquert, die darin enthaltene Feuchtigkeit verdampft und ohne Schädigung der nützlichen Bodenbakterien die schädlichen tierischen Lebewesen bzw. Unkrautsamen vernichtet. Die Durchlaufzeit so kann durch Änderung der Trommelneigung eingestellt werden. Eine weitere Regelung ist durch die Ölzufuhr zum Brenner und die Änderung der Drehgeschwindigkeit der Trommel möglich.

In Weiterbildung hierzu ist aus der DE1115511B eine Maschine zur Erddämpfung mit einer drehend angetriebenen, von heißen Gasen in Längsrichtung durchströmten, geneigt angeordneten und an ihrem inneren Umfang mit Vorsprüngen, Schaufeln u. dgl. versehenen Trommel und einer Zerkleinerungseinrichtung für die in dem Gut vorhandenen Erdknollen bekannt, bei der die hocherhitzbare Zerkleinerungseinrichtung am Austrittsende der Trommel angeordnet ist. Weiterhin ist an ihrem Austrittsende die Trommel als Siebtrommel ausgebildet und mit dem unteren Teil der Siebtrommel wirkt die hierzu relativ bewegte Zerkleinerungseinrichtung für die Erdknollen zusammen. Dies ist beispielsweise eine schneller angetriebene Walze, ein hin und her bewegtes zweites Sieb oder eine ähnlich wirkende Einrichtung zur Zerkleinerung der Erdknollen. Die vorher hauptsächliche Zerkleinerung des behandelten Erdgutes erfolgt schon mit dem Dämpfen und Sterilisieren während des Durchlaufes durch die sich drehende Trommel, welche die Erde dauernd anhebt und zwingt, durch die heißen Gase zu fallen, welche die Trommel durchströmen. Eventuell noch verbleibende Erdknollen müssen zuvor in der am Trommelende angebrachten Zerkleinerungseinrichtung zerkrümelt werden, wobei sie durch die innige Berührung mit den Zerkleinerungswerkzeugen, welche durch Wärmestrahlung von der Flamme und Wärmeleitung von den übrigen Teilen der Trommel her eine ausreichend hohe Temperatur aufweisen, ebenfalls sterilisiert werden. Dazu kommt noch die beim Austritt aus der Trommel bewirkte Vermischung dieser Teilchen mit der weitaus überwiegenden Menge der schon beim Durchgang durch die Trommel bereits zerkleinerten und durch die heißen Gase erhitzten Erdkrumen bzw. Erdzerkleinerung.

In Weiterbildung hierzu ist schließlich aus der CH682203A5 eine Vorrichtung zur Hitzebehandlung von Kulturerde bekannt, bei der das gesamte behandelte Erdgut in fein krümeliger Struktur aus der Trommel austritt und keine unzerkleinerten Knollen verbleiben, deren Inneres dann naturgemäß nicht in gleicher Weise der Hitzebehandlung unterworfen würden und bei der das feuchte Erdgut nicht stellenweise an den inneren Teilen der Trommel festklebt und nicht durch die Vorrichtung hindurchgefördert wird. Hierzu weist die Vorrichtung mehrere Siebetagen mit auf jeder Siebetage drei Siebe mit unterschiedlich grober Lochung hintereinander auf. Dabei wird das vorzerkleinerte, auf die oberste Siebetage aufgegebene Erdgut durch Presswalzen in der obersten Etage und dem Sieb entknollt und aufgekrümmelt, worauf es je nach Größe der Siebmaschen und Neigung der Rüttelsiebe relativ langsam und zeitlich kontrolliert als "Erdregen" durch sämtliche Siebetagen durchgearbeitet wird, dabei jedes Mal gebremst und der Einwirkung der Dämpfflamme umso länger ausgesetzt wird. Ein Gebläse für die erforderliche Sekundärluft ist seitlich des Behandlungsraumes auf einem Rahmen angebracht. Ein verstellbares Abschirmblech verhindert das Austreten des geworfenen Erdgutes. Die erzeugte Hitze von 400°C bis 600°C steigt durch den Siebraum auf und kann durch in einer Verschalung angebrachte verstellbare Klappen entweichen. Dabei kann ein zusätzliches Gebläse mithelfen, eine gleichmäßige Durchdringung zu erreichen. Brennerleistung, Siebneigung und Sekundärluft können über eine elektronische Steuervorrichtung regulierbar sein. Von Kreuzwalzen wird das gedämpfte Erdgut nochmals intensiv durchkrümelt und zwar noch im heißen Dämpfraum - eine weitere Verzögerung des Durchlaufs - und von der darunter befindlichen Rüttelwanne aufgefangen und abgeführt.

Weiterhin ist aus dem DE20207214U1 ein Trommelheizer mit Drehtrommel und Heißgasbrenner zum Einblasen von Heißgasen in den mit zumindest einem Heißgasleitsegment versehenen Drehtrommelinnenraum, insbesondere zum Asphaltrecycling bekannt. Durch die Anwendung der indirekten Heißgasbeheizung ist einerseits eine schnelle und wirtschaftliche Einsatzbereitschaft gewährleistet und andererseits eine umweltschonende Behandlung ohne Freisetzung von Materialverbrennungs- oder Crackgasen ermöglicht. Dabei ist es durch die indirekte Beheizung möglich, Übertemperaturen und Materialverbrennungen im zu recycelnden / zu behandelnden Material zu vermeiden. Jedoch wird bei diesem technisch entfernten Gebiet des Asphaltrecycling das kontinuierlich anfallende Asphaltgranulat auf eine ca. 130°C bis 170°C liegenden Endtemperatur erhitzt und ist auch nach Erreichen dieser Temperaturen noch rieselfähig. Im Einzelnen ist innerhalb des Drehtrommelinnenraumes ein ein- oder mehrteiliges Heißgasleitsegment angeordnet, das zumindest eine Heißgasprallfläche aufweist, die sich von der Drehtrommelinnenwand zum Drehtrommelinneren hin erstreckt und gegen die Heißgasströmrichtung ausgerichtet ist. Das Heißgas wird vorzugsweise im Wesentlichen parallel zur der Dreh-/Längsachse der Drehtrommel eingeblasen. Die gegenüberliegende Seite der Drehtrommel weist eine Beschickungsöffnung auf, an der eine Vorrichtung zur Materialzufuhr, vorzugsweise in Form einer trichterartigen Rutsche, angebracht ist. Das erwärmte Gut, z.B. der zerkleinerte bzw. teilaufgeschmolzene Asphalt, verlässt die Drehtrommel im Bereich des dem Heißgasbrenner zugewandten Drehtrommelendes durch eine oder mehrere vorzugsweise verschließbare Öffnungen in der Drehtrommelwand. Um eine gute Durchmischung und auch eine Förderwirkung zu erhalten, ist die Drehtrommel im Betriebszustand in wesentlichen waagerecht angeordnet. Durch Absenken des Drehtrommelendes kann die Drehtrommel in eine bestimmte Neigung versetzt werden, wobei hiermit die Verweildauer des Mischgutes in der Drehtrommel reguliert wird. Dies kann, soweit die Drehtrommel auf einem Fahrzeugchassis montiert ist, am einfachsten durch einseitiges Absenken des Fahrzeugchassis bewirkt werden. Durch die Anordnung der als Wärmestau- und Leitsegmente wirkenden Heißgasleitsegmente im Drehtrommelinnenraum wird die Wärme besser im Drehtrommelinnenraumes umgelenkt, verteilt und gestaut, so dass die äußeren Bereiche des Drehtrommelinnenraumes besser mit Wärme versorgt werden, ohne dass die sich bevorzugt dort aufhaltenden Materialbrocken direkt mit der Brennerflamme in Kontakt kommen. Die Heißgasprallflächen sind als Viertel- bis Drittelkreisflächen ausgebildet, die gegenseitig in gleichbleibender Versetzungsrichtung und Winkelgraden gleichmäßig versetzt angeordnet sind. Außer der Heißgas-Umlenkung im Drehtrommelinnenraum kann auch die Verweildauer der Asphaltbrocken zwischen den einzelnen Segmenten gesteuert werden. Während des Durchlaufes nehmen die Asphaltbrocken die Wärme auch von den Heißgasprallflächen auf und zerfallen in kleinere Korngrößen. Das dabei entstehende Asphaltgranulat durchläuft den Drehtrommelinnenraum im Wesentlichen im unteren Bereich der Drehtrommel und von der Brennerflamme unerreicht. Die erforderliche Asphalt- Einbautemperatur erreicht das Mischgut durch Wärmeabgabe der erhitzten Drehtrommelwandung und durch Kontakt mit dem umgelenkten Heißgas, aber ohne direkten Brennerflammenkontakt.

Um die Kosten zu senken, kommen in kleinerem Rahmen in Gärtnereien und Baumschulen Erdedämpfgeräte (auch Erddämpfgeräte) zum Einsatz. Sie sind in der Form von Schubkarren mit Deckel und Dämpfeinheit erhältlich, aber auch als kleine Dämpfwagen. Der komplette Schubkarrendämpfer besteht aus einer Schubkarre mit Dampfererzeuger, Prüfthermometer, Gitterrost, Jutefilter und Deckel. Die erreichbare Dämpftemperatur liegt bei 80 Grad Celsius und erfüllt somit die Mindestanforderung, um Krankheitserreger, Schädlinge, Pilzsporen und Unkrautsamen unschädlich zu machen. Die Beheizung erfolgt elektrisch und die Dämpfzeit beträgt etwa 10 Minuten. Gleiches Ergebnis kann bei einer Schubkarre mit einem auf halber Höhe waagrecht eingesetzten luftdurchlässigen Zwischenboden (beispielsweise Lochblech), wobei das eine Ende der Dampfleitung unter den Zwischenboden geschoben wird, erzielt werden.

In ähnlicher Weise ist ein Gerät zum Sterilisieren von Erde mittels Dampf aus der DE0805204B ausgestaltet, welches als Kleingerät sich auch für mittlere und kleinere Betriebe eignet und äußerst einfach zu bedienen ist. Das Gerät besteht aus einem zylindrischen Kessel, der in einem Gestell kippbar aufgehängt ist und in beliebiger, an sich bekannter Weise z. B. mittels einer am Gestell befestigten Bogenführung und einer am Kessel festen Schraube mit Flügelmutter in jeder Schräglage festgestellt werden kann. Der Kessel wird oben durch einen Deckel verschlossen, der in beliebiger an sich bekannter Weise angepresst wird. Ein den Deckel durchdringendes Rohr mit Kappe wirkt als Sicherung gegen etwaigen Überdruck. Ferner enthält der Deckel eine Öffnung zum Einführen eines Kontrollthermometers. Kessel und Deckel sind doppelwandig mit einer wärmeisolierenden Zwischenschicht, beispielsweise aus Glaswolle, ausgeführt. Am Boden des Kessels befindet sich die Heizvorrichtung, und zwar vorzugsweise eine elektrische. Darüber befindet sich der Raum, welcher das zu verdampfende Wasser aufnimmt und einen nach außen führenden Rohrstutzen mit Ablass- und Probierhahn aufweist. Dieser Wasserraum ist gegen den darüberliegenden Raum so abgeschlossen, dass kein Wasser in die zu entkeimende Erde gelangen kann. Von dem Wasserraum führen mehrere Dampfrohre, welche gleichmäßig über den Querschnitt des Kessels verteilt sind, bis nahe an das obere Kesselende. Die Dampfrohre sind oben geschlossen und haben über ihre Länge verteilt Löcher, so dass der Dampf die Erde nicht von unten nach oben durchströmt, sondern in vielen übereinanderliegenden Schichten austritt. Die untersten Dampfaustrittslöcher dürfen nicht zu tief liegen, damit durch sie kein mitgerissenes Wasser aus dem Wasserbehälter in die Erde gelangen kann. Es empfiehlt sich, den ursprünglich runden Querschnitt der Rohre abzuflachen, was eine Raumersparnis ohne wärmetechnische Nachteile mit sich bringt. Das Kontrollthermometer, welches durch ein Loch im Deckel eingeführt werden kann, ist ein nach Art einer Heustocksonde gebautes Rohr mit einem Thermoelement am unteren Ende und einer Ablesevorrichtung am oberen Ende. Mit dem Kontrollthermometer kann man sich jederzeit überzeugen, ob in allen Schichten der Erdfüllung die gewünschte Temperatur erreicht ist.

Um eine Gleichmäßigkeit der Sterilisierung der Erde zu erreichen und um den Kompost im Hobbygarten professionell zu entkeimen ist aus der DE202020002398U1 ein Betonmischer zur Kompostbehandlung bekannt, bei dem unterhalb der Mischtrommel des Betonmischers ein Gasbrenner angeordnet ist. Der Gasbrenner wird mit Flüssiggas, Erdgas oder anderen Brenngase betrieben und die Mischtrommel kann mit einen Deckel verschlossen werden. Der Verschlussdeckel oder die Mischtrommel ist mit einer Temperaturmesseinrichtung zur Anzeige der Temperatur versehen. Nur bei laufender Mischtrommel des Betonmischers kann der Gasbrenner in Betrieb genommen werden und bei Stillstand der Mischtrommel oder nach Erreichen der Abschalttemperatur oder bei einer Lageänderung wird der Gasbrenner wieder außerbetrieb genommen.

Schließlich ist aus der DE 42 39 771 C1 eine Vorrichtung zur thermischen Behandlung von Schüttgut bekannt. Im Einzelnen weist die Vorrichtung eine flächige Aufnahmeeinrichtung, auf welche das Schüttgut aufgegeben wird, und eine über der Aufnahmeeinrichtung angeordnete Brennereinrichtung, durch welche das Schüttgut auf der Aufnahmeeinrichtung direkt beflammt wird. Das Schüttgut der Aufnahmeeinrichtung wird über einen Schacht mit einer oder mehreren Siebetagen zugeführt und die durch die Brennereinheit erzeugte Heißluft wird zumindest größtenteils durch den Schacht abgeführt. Der Schacht ist dabei über der Aufnahmeeinrichtung und im Wesentlichen senkrecht angeordnet und die Heißluft steigt thermisch in dem Schacht auf. Die Siebetagen weisen im Wesentlichen waagrechte, übereinander im Abstand zueinander angeordnete Siebböden auf und die Heißluft wird im Wesentlichen im Querstrom waagrecht zwischen den Siebetagen hindurchgeführt. In zwei einander gegenüberliegenden Wänden des Schachtes sind Luftführungen angeordnet, die jeweils die Räume zwischen zwei aufeinanderfolgenden Siebetagen miteinander verbinden, wobei die Luftführungen der einander gegenüberliegenden Wände des Schachtes jeweils um eine Siebetage vertikal gegeneinander versetzt sind. Die Siebböden weisen für das Schüttgut undurchlässige Flächenbereiche auf, welche in Querstromrichtung der Heißluft verlaufende, über die Breite der Siebböden durchgehende Streifen sind. Die undurchlässigen Flächenbereiche sind vertikal aufeinanderfolgender Siebböden, welche jeweils gegeneinander versetzt angeordnet sind. Der Mantel des Schachtes ist wärmeisoliert und die Siebetagen sind als Schwing- oder Schüttelsiebe ausgebildet. Insbesondere weisen die Siebetagen fest in den Schacht eingesetzte Siebböden auf und der Schacht ist insgesamt in schwingende bzw. schüttelnde Bewegung versetzbar. Das durch die unterste Siebetage fallende Schüttgut gelangt auf eine geneigte Stufenplatte und rutscht auf dieser herab, was wiederum durch einen Schwingungserzeuger begünstigt wird. Beim Herabrutschen auf der Stufenplatte gelangt das Schüttgut unter das Flächenraster der Brennerdüsen eines Gasbrenners, wird flächig direkt beflammt und auf die zur Sterilisation erforderliche Temperatur erhitzt. Das Durchströmen des Schüttgutes im Querstrom und das Ansammeln des Schüttgutes auf den undurchlässigen Bereichen der Siebböden führt zu einer langen Kontaktdauer zwischen der Heißluft und dem Schüttgut, so dass ein sehr intensiver Wärmeaustausch von der Heißluft auf das Schüttgut erfolgt. Die Heißluft gibt dabei auf ihrem Weg in dem Schacht nach oben zunehmend ihre Wärme ab, während sich das Schüttgut auf seinem Weg in dem Schacht von oben nach unten zunehmend erwärmt.

Wie die vorstehende Würdigung des Standes der Technik aufzeigt, sind unterschiedliche ausgestaltete Vorrichtungen zur thermischen Behandlung von Schüttgut bekannt. Dabei ergeben sich jedoch spezielle Randbedingungen aus dem thermisch zu behandeltem Schüttgut. Insbesondere fehlt in der Praxis eine kostengünstige und unter dem Gesichtspunkt Energieeffizienz (annähernd gleiche Temperatur) und Durchlaufzeiten optimierte Vorrichtung, welche einen kontinuierlichen Arbeitsablauf mit wenig Personal ermöglicht und ein Sicherheitsrisiko für den Anwender zuverlässig vermieden wird.

Der Erfindung liegt die Aufgabe zugrunde, eine Vorrichtung zur thermischen Behandlung von Schüttgut derart auszugestalten, dass diese eine kontinuierliche und energieeffiziente Beschickung ermöglicht und ein Sicherheitsrisiko für den Anwender zuverlässig vermeidet wird.

Diese Aufgabe wird, gemäß Patentanspruch 1, durch eine Vorrichtung zur thermischen Behandlung von Schüttgut gelöst, welche aufweist:
- ein mehrstöckiges Gestell aus Rohrrahmen, in welchem im untersten Stock am unteren Tragrahmen ein Trommelkörper mit Mischschaufeln gelagert und auf dem im Abstand darüber liegenden Tragrahmen im obersten Stock ein Rüttelsieb angeordnet ist,
- eine Rutsche zur Zuführung des vorgesiebten Mischguts zu einer Trommelöffnung des Trommelkörpers,
- einen außerhalb des Trommelkörpers angeordneten Gasbrenner, dessen Flamme sich in das Innere des Trommelkörpers erstreckt und
- ein im Innern des Trommelkörpers oberhalb der Flamme angeordnetes Prallblech.

Weiterhin wird diese Aufgabe, gemäß Patentanspruch 13, durch ein Verfahren zur thermischen Behandlung von Schüttgut für eine Vorrichtung nach Anspruch 1 gelöst, bei dem:
a) das Schüttgut einem im obersten Stock eines mehrstöckigen Gestells angeordneten Rüttelsieb zugeführt wird,
b) das vorgesiebte Mischgut in einen Trommelkörper eingefüllt wird,
c) in dem Trommelkörper von der offenen Flamme eines Gasbrenners im Inneren des Trommelkörpers aufgeheizt wird und
d) das erhitzte Mischgut mittels schraubenlinienförmig zueinander angeordnete Mischschaufeln vom Boden der Mischtrommel nach oben und zur Auslassöffnung des Trommelkörpers gefördert wird.

Die Vorrichtung und das Betriebsverfahren gemäß der Erfindung weisen den Vorteil auf, dass nur ein Benutzer erforderlich ist, um das eingebrachte Schüttgut zu sieben und kontinuierlich innerhalb kürzester Zeit keimfrei zu machen.

Weitere Vorteile und Einzelheiten lassen sich der nachfolgenden Beschreibung von bevorzugten Ausführungsformen der Erfindung unter Bezugnahme auf die Zeichnung entnehmen. In der Zeichnung zeigt:
- Fig. 1: in Seitenansicht, teilweise im Schnitt eine Ausgestaltung der Vorrichtung gemäß der Erfindung,
- Fig. 2: in perspektivischer Ansicht und Seitenansicht die Vorrichtung nach Fig. 1, teilweise im Schnitt,
- Fig. 3: in perspektivischer Draufsicht die Vorrichtung nach Fig. 1,

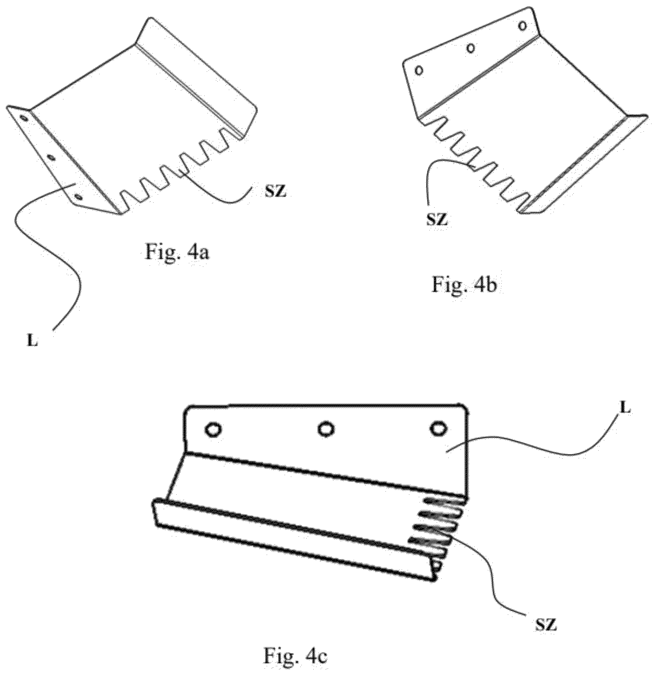

- Fig. 4a, 4b und Fig. 4c: perspektivische Ansichten einer Ausgestaltung der Mischschaufeln,
- Fig. 5: in perspektivischer Ansicht die Lagerung des Rüttelsiebs auf Federn,
- Fig. 6: in Seitenansicht, teilweise im Schnitt eine Ausgestaltung des Aufbaus der Vorrichtung auf einem Fahrzeuganhänger mit Anhängerkupplung und Fahrzeugdeichsel,
- Fig. 7: in Seitenansicht die Vorrichtung nach Fig. 6 mit ausgefahrenen Klappstützen,
- Fig. 8: in perspektivischer Ansicht und Seitenansicht die Vorrichtung im Detail mit trennbarer Förderschnecke, Laufsteg und Hydraulikantriebsmotor für Erregerwelle,
- Fig. 9: in Seitenansicht die Vorrichtung im Detail mit Benzin-/ Dieselmotor,
- Fig. 10: in Seitenansicht die Vorrichtung im Detail mit Trommelisolierung,
- Fig. 11: in perspektivischer Ansicht und im Detail die Erregerwelle und den Hydraulikantriebsmotor,
- Fig. 12: in Seitenansicht und im Detail die Abgasheizung für Gasflaschen,
- Fig. 13: in perspektivischer Ansicht und im Detail die Vorrichtung mit Transportsicherung und
- Fig. 14: in perspektivischer Ansicht die erfindungsgemäße Vorrichtung mit zusätzlichem Brenner an der Auslassöffnung.

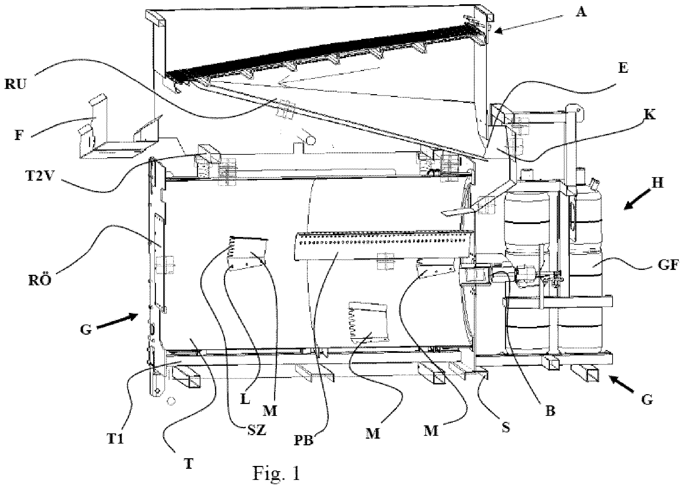

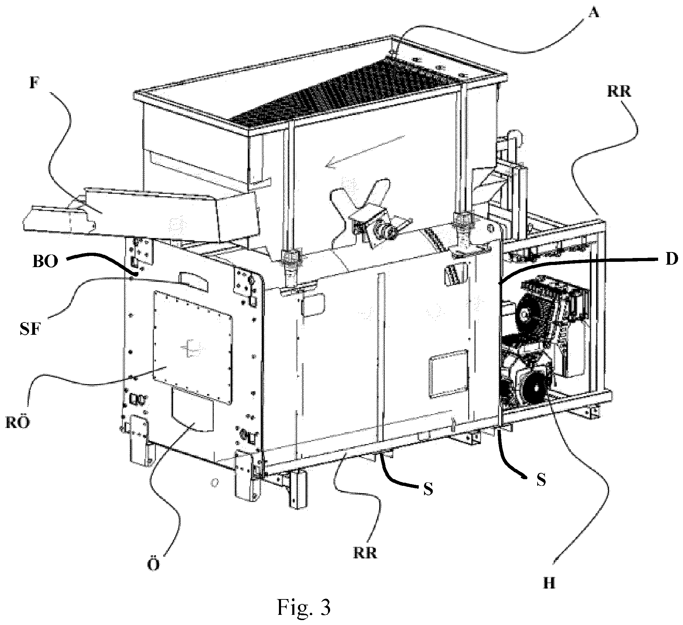

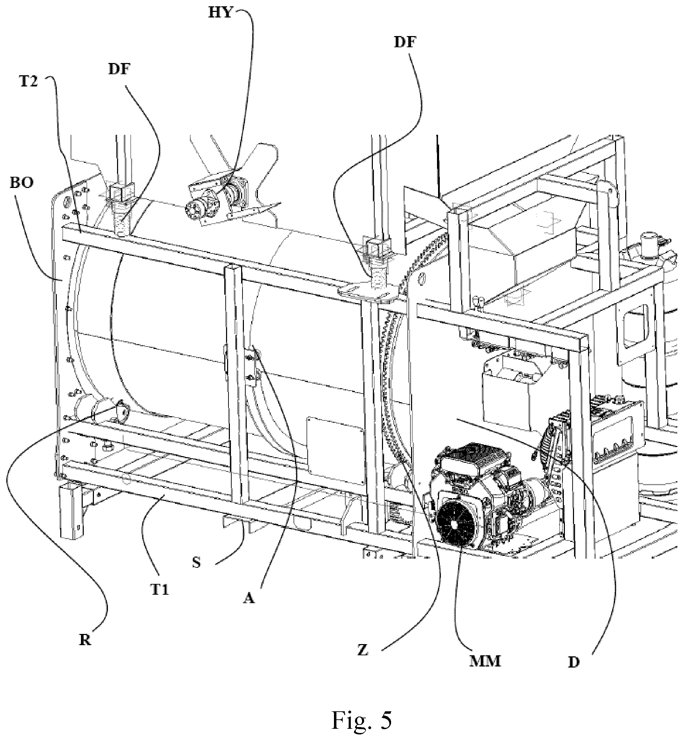

Fig. 1 bis Fig. 3 zeigen eine Ausgestaltung der Vorrichtung gemäß der Erfindung zur thermischen Behandlung von Schüttgut, insbesondere von Erde, nachfolgend auch Mischgut genannt. Im Einzelnen weist diese ein mehrstöckiges Gestell G aus Rohrrahmen RR auf, in welchem am unteren Tragrahmen T1 ein Trommelkörper T mit Mischschaufeln M gelagert ist. Auf dem im Abstand darüber liegenden Tragrahmen T2 ist ein Rüttelsieb A angeordnet. Das Rüttelsieb A ist auf Federn, insbesondere Druck- oder Tellerfedern gelagert, damit der darunter liegende Trommelkörper T beim Betrieb des Rüttelsiebs A nicht mitvibriert (siehe Fig. 5).

Weiterhin weist die Vorrichtung eine Rutsche RU zur Zuführung des vorgesiebten Mischguts zu einer Trommelöffnung E des Trommelkörpers T, einen außerhalb des Trommelkörpers T angeordneten Gasbrenner B, dessen Flamme sich in das Innere des Trommelkörpers T erstreckt und ein im Innern des Trommelkörpers T oberhalb der Flamme angeordnetes Prallblech PB auf.

Die durch das Rüttelsieb A gesiebte Erde wird über die Rutsche RU zur Einfüllöffnung E der Mischtrommel T bewegt. An der Einfüllöffnung/Trommelöffnung E der Mischtrommel T ist eine Feder bzw. Gasdruckfeder unterstützte Klappe K angebracht auf diese das Siebgut /Erde rutscht. Bei einem voreingestellten Wert (z.B. 5 kg) öffnet sich die Klappe K und lässt das Siebgut in die Trommel T. Danach schließt die Klappe K wieder. Diese Klappe K verhindert weitgehend, dass die heiße Luft in der Trommel T nicht über die Einfüllklappe K entweichen kann. Somit wird weniger Energie verbraucht, da die heiße Luft in der Mischtrommel T verbleibt.

Das Prallblech PB wird direkt über dem Brennereintritt platziert. Damit wird die Brennerflamme bzw. die Abgase weiter in die Trommelmitte geführt. Da sich die Einfüllöffnung E des Siebgutes direkt über dem Brennereintritt in die Mischtrommel T befindet, wird durch das Prallblech PB ein Durchfallen des gesiebten Schüttgutes/Erde direkt durch die Flamme verhindert.

Der Trommelkörper T ist zylindrisch, erstreckt sich auf dem unteren Tragrahmen T1 im Wesentlichen horizontal und weist im benachbarten Deckel D die Öffnung für die Flamme und im benachbarten Boden BO eine Öffnung Ö für den Auslass des Schüttguts aufweist. Der untere Tragrahmen T1 weist Räder auf, so dass die Vorrichtung auch mobil betrieben werden kann. Weiterhin sind am Gestell G Staplerlaschen S zum Anheben und Bewegen der Vorrichtung angeordnet.

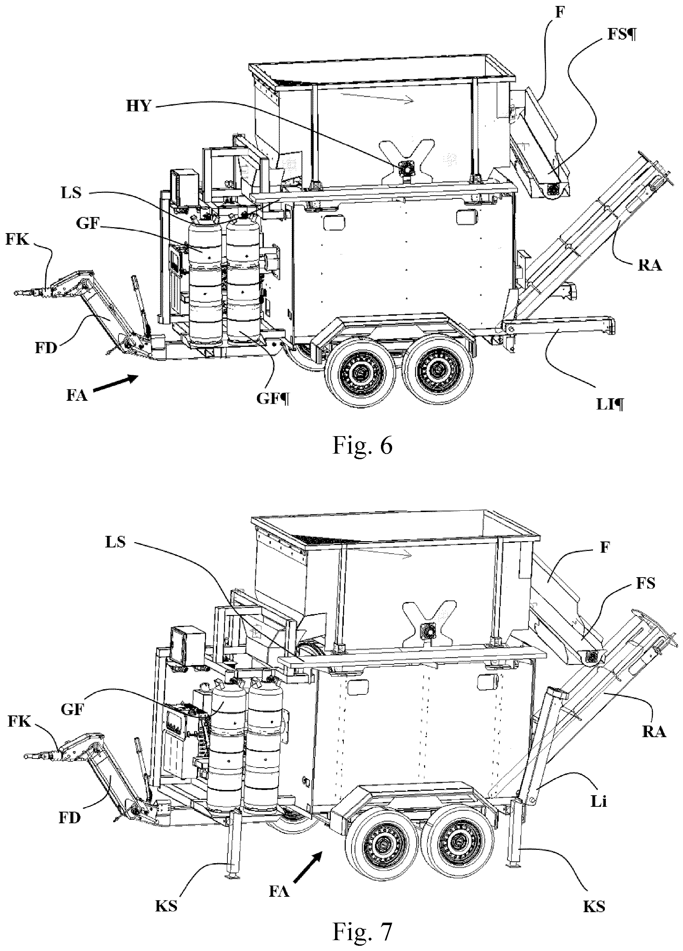

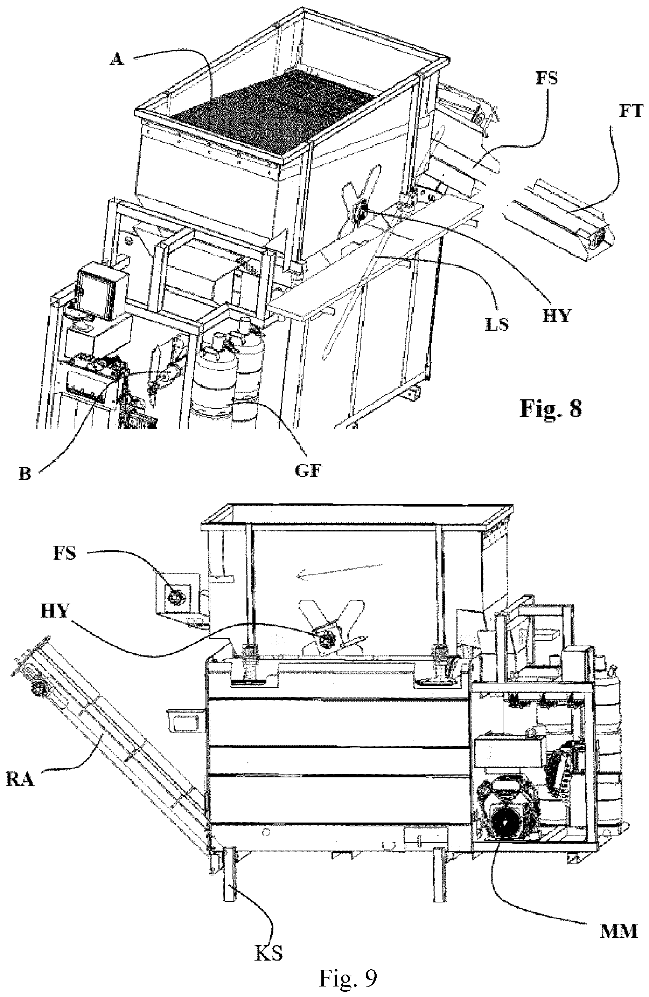

Insbesondere zum mobilen Betrieb ist die Vorrichtung auf einem Fahrzeuganhänger FA mit Anhängerkupplung FK und Fahrzeugdeichsel FD aufgebaut, welcher im Detail in Fig. 6 bis Fig. 14 dargestellt ist. Der Fahrzeuganhänger FA hält dabei die gesetzlichen Bestimmungen, insbesondere hinsichtlich Breite (max. 2,55m) ein. Das Modul wiegt zwischen 2t und 3,5 t, wobei die Förderschnecke FS für das nach Korngröße gesiebte Grobgut/Überkorn mit einem trennbaren Vorderteil FT oder einem klappbaren Förderband F (in der Zeichnung nicht dargestellt) ausgestaltet ist. Im Hinblick auf die Gesamtlänge des Fahrzeuganhängers FA ist dieser mit klappbaren Lichterblenden LI versehen und für den Fahrbetrieb wird zwischen den Lichterblenden LI ein Förderband RA angeordnet, welches (siehe Fig. 6, 7 ,9, 10, 11, 13) vor der Auslauföffnung Ö angeordnet ist und im stationären Betrieb das thermisch behandelte Schüttgut kontinuierlich beispielsweise in einen Container, Baustoffbehälter befördert.

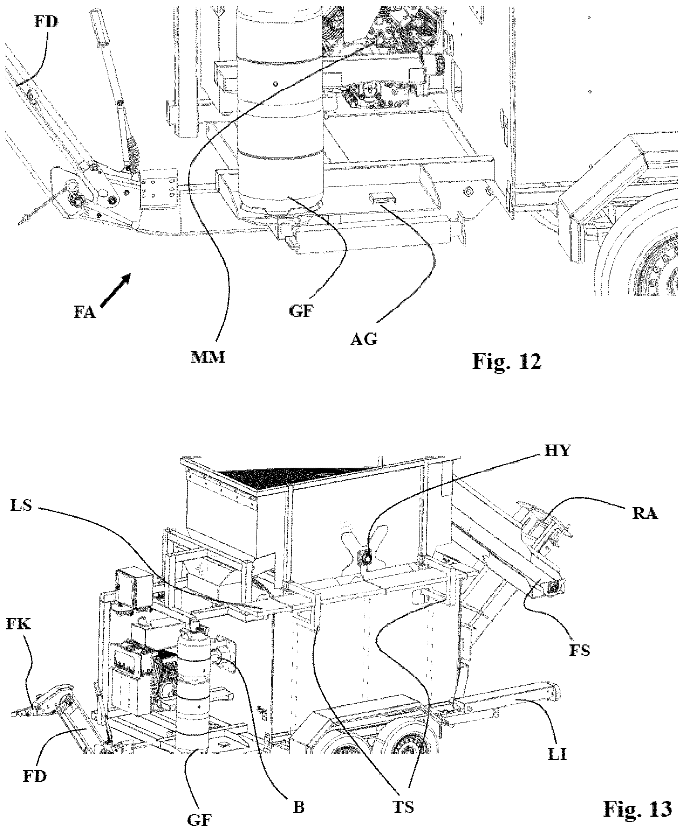

Entsprechend mittels der Förderschnecke FS das Überkorn zu trennen (trennbares Vorderteil FT) und den klappbaren Lichterblenden LI kann die Vorrichtung mobil auf einem Fahrgestell/Fahrzeuganhänger FA aufgebaut werden und mit den Transportsicherungen TS an den Einsatzort transportiert werden (siehe Fig. 1 Verriegelungsöffnung T2V (Rohrrahmen RR des Gestells G) und Fig. 13 mit U-förmigen Sicherungsbügel, welcher den oberen Tragrahmen T2 sichert. Am Einsatzort kann die Anlage von einer Bedienperson einsatzbereit gemacht werden.

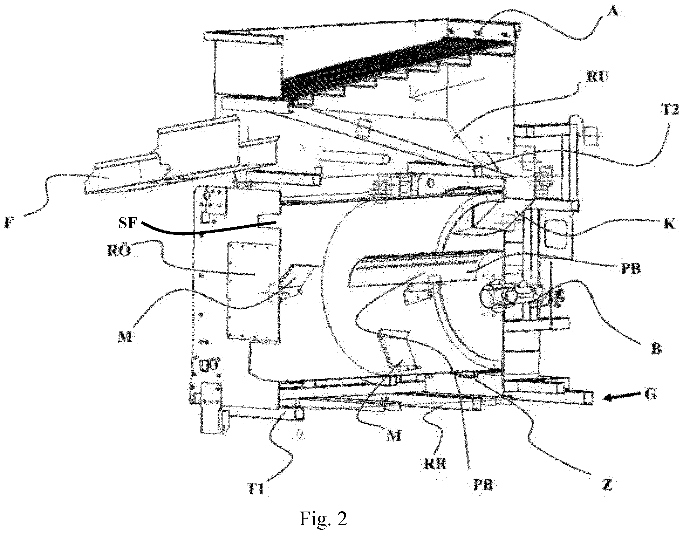

Eine noch energiesparende Aufheizung des Schüttguts/Mischmaterials wird durch eine isolierte Mischtrommel TI ermöglicht (siehe Fig. 2 oder Fig. 5 oder Fig. 10 mit entfernter Verkleidung).

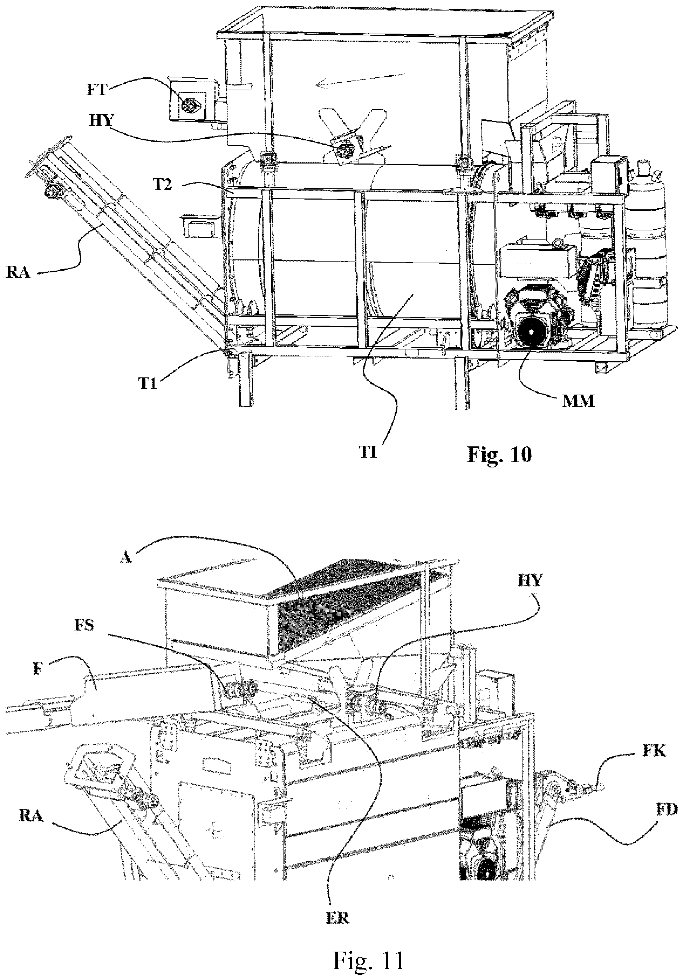

Die Erregerwelle ER (siehe Fig. 11) angetrieben durch einen stufenlos regelbaren Hydraulikmotor HY (siehe Fig. 5, Fig. 6, Fig. 8, Fig. 9, Fig. 10, Fig. 11, Fig. 13, Fig. 14) versetzt das Rüttelsieb A drehzahlabhängig in Schwingung. An der Erregerwelle ER sind verstellbare Gewicht angebracht mit der die Intensität der Erregerwelle ER eingestellt werden kann. Beispielsweise sind verstellbare Unwuchtscheiben vorgesehen, welche an beiden Wellenenden verstellbar sind. Somit kann das Arbeitsmoment, die Fliehkraft und die Schwingbreite angepasst werden.

Insbesondere im Winter werden mit einem Abgasführungssystem AG die Gasflaschen GF von unten mit warmen Abgasen vom Antriebsmotor MM ummantelt. Dies verhindert eine zu schnelle Vereisung der Gasflaschen GF.

Durch die Klappstützen KS kann der Fahrzeuganhänger FA am Einsatzort stabil abgestellt werden, um unnötige Schwingungen des Moduls/der Vorrichtung zu verhindern.

Insgesamt kann die Vorrichtung als Modul auf einem Fahrgestell des Fahrzeuganhängers FA aufgebaut und mit Transportsicherungen TS für das Rüttelsieb A an den Einsatzort transportiert werden. Am Einsatzort kann diese dann von einer Bedienperson wieder einsatzbereit gemacht werden.

Der Trommelkörper T selbst ist freilaufend auf mehreren Rollen R in der Vorrichtung am unteren Tragrahmen T1 gelagert und rotiert um seine zentrale Achse. In der Nähe des Deckels D (Abdeckblech stirnseitig) ist am Mantel des Trommelkörpers T in Querrichtung umlaufend ein Antriebsmittel Z angeordnet. Vorzugsweise ist das Antriebsmittel Z ein am Mantel angeschweißter Zahnkranz.

Auf der Innenseite des Trommelkörpers T sind im Abstand zueinander die Mischschaufeln M angeordnet, welche im wesentlichen U-förmig ausgestaltet sind. An einem der Schenkel jeder Mischschaufel M ist eine Lasche L angeordnet, welche an die jeweilige schraubenlinienförmig zueinander angeordnete Befestigungsvorrichtung an der Innenseite der Trommel T form oder kraftschlüssig befestigt ist. Die Basis der Mischschaufeln M ist auf einer Seite (in Förderrichtung) vorzugsweise sägezahnförmig SZ ausgestaltet. Auf der gegenüberliegenden Seite des Sägezahn SZ kann auch eine Abkantung der Basis der Mischschaufeln M vorgesehen werden, wodurch die gezielte fächerförmige Verteilung der Erde unterstützt wird.

Der Gasbrenner B weist einen Flammenwächter auf, welcher die Ausbildung der Flamme überwacht und regelt. In der Praxis wird vorzugsweise eine ca. 50 cm Flamme erzeugt, wobei das oberhalb befindliche Prallblech PB eine Länge von ca. 80 cm aufweist. Das Prallblech PB ist insbesondere kreissegmentförmig oder keilförmig gebogen und ist am Blechdeckel D befestigt, vorzugsweise angeschraubt, so dass dieses einfach zur Reinigung herausgenommen oder auszutauschen ist.

Der Trommelkörper M besteht aus einzelnen abgekanteten Teilabschnitten, welche stoffschlüssig miteinander verbunden sind und die am Mantel umlaufende(n) Abkantung(en) AK werden in einem am Gestell G angeordneten Führungsmittel geführt. An der Stirnseite (auch als Boden BO bezeichnet) der Vorrichtung weist der Boden BO eine (rechteckige) Revisionsöffnung RÖ, eine darunter liegende Auslassöffnung Ö und ein darüberliegendes Sichtfenster SF auf. Das Sichtfenster SF dient insbesondere als Zugöffnung (einstellbar durch eine verschiebbare, insbesondere Leisten geführte Blechabdeckung) für die Regelung der Temperatur, welche an einem neben dem Sichtfenster SF angeordneten Thermometer (in der Zeichnung nicht dargestellt) abgelesen werden kann. Die dekontaminierte Erde fällt aus dem offenen Trommelkörper über Bleche geführt an der Auslassöffnung Ö heraus.

Wie die vorgehende Beschreibung aufzeigt, wird im vorliegenden Anwendungsfall die Vorrichtung dazu benutzt, die eingebrachte Erde innerhalb kürzester Zeit keimfrei zu machen.

Mittels Gasbrenner B wird die Trommel T von innen kontinuierlich beflammt. Die Trommel T sowie das aufgesetzte wechselbare Rüttelsieb A sind in einem stabilen Rohrahmen RR untergebracht. Die Trommel T ist freilaufend auf vier Rollen R gelagert und wird über einen Zahnkranz Z von außen hydraulisch stufenlos angetrieben.

Bei dem Aufheizprozess wird die Erde auf ca.80°C erwärmt, was nahezu alle Keime zerstört. Die über ein Rüttelsieb A vorgesiebte Erde rutscht über eine Rutsche RU in die Trommelöffnung E der Mischtrommel T.

Durch die Mischschaufeln M wird die Erde vom Boden der Mischtrommel T nach oben gefördert. Bei diesem Vorgang rieselt die Erde ab einer bestimmten Höhe von den Mischschaufeln M und fällt durch die Brennerflamme bzw. deren Abgase und dem Prallblech PB wieder auf den Trommelboden. Durch die Anordnung der Mischschaufel M wird die Erde kontinuierlich in Richtung Auslauf / Auslassöffnung Ö gefördert.

Trommel T, Förderanlage F (Förderband , Förderschnecke FS einschließlich trennbares Vorderteil FT für Transport) und Rüttelsieb A werden über eine mit Benzin- oder Dieselmotor betriebene Hydraulikanlage H betrieben.

Zur Beschickung des Rüttelsiebs A kann ein Förderband oder ein Bagger (Radlader) verwendet werden. Dabei weist die Vorrichtung einen im Bereich der des obersten Stocks angeordnete Laufsteg LS auf (siehe Fig. 6, 7, 8, 13).

DieFörderanlage F dient zum Abfördern des nach Korngröße gesiebten Grobguts. Durch den schmalen Spalt zwischen Trommelkörper M und Boden BO und Deckel D kann der beim "Kochen der Erde" entstehende Wasserdampf entweichen.

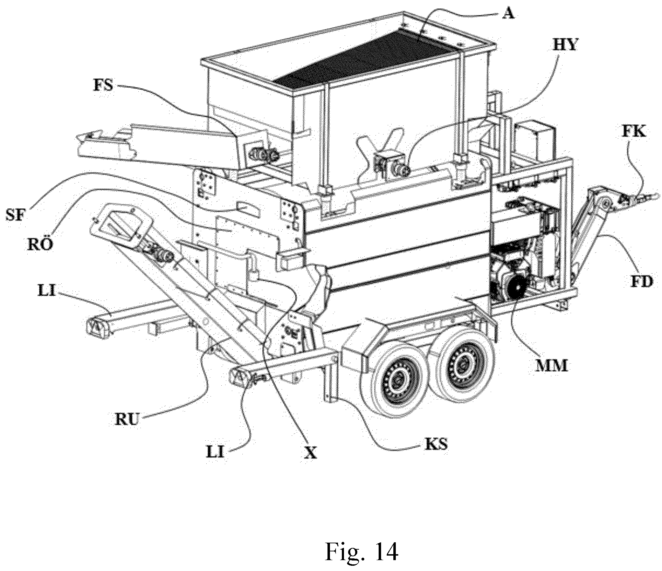

Schließlich ist - wie Fig. 14 zeigt - an der Auslassöffnung Ö ein zusätzlicher Brenner X angeordnet. Insbesondere für feuchtere Erde, welche kontinuierlich in Richtung Auslauf / Auslassöffnung Ö gefördert wird, kann deren Krümmeln/Brockenbildung durch größere Wärmeinwirkung reduziert werden.

Die Erfindung ist nicht auf die dargestellten und beschriebenen Ausführungsbeispiele beschränkt, sondern umfasst auch alle im Sinne der Erfindung gleichwirkenden Ausführungen der erfindungsgemäßen Vorrichtung, d.h. die Erfindung ist nur durch die Patentansprüche beschränkt.

### Bezugszeichenliste:

- A: Rüttelsieb (auf Federn (Druck- oder Tellerfedern) gelagert)
- AG: Abgasheizung für Gasflaschen
- AK: Abkantung (Mantel von T)
- B: Gasbrenner
- BO: Boden (Stirnseite der Vorrichtung)
- D: Deckel (Stirnseite der Vorrichtung)
- DF: Druck- oder Tellerfedern
- E: Trommelöffnung
- ER: Erregerwelle
- F: Förderband/anlage (Abfördern des nach Korngröße gesiebten Grobguts)
- FA: Fahrzeuganhänger (mit Anhängerkupplung und Fahrzeugdeichsel)
- FD: Fahrzeugdeichsel
- FK: Anhängerkupplung
- FS: Förderschnecke
- FT: Förderschnecke, trennbares Vorderteil
- G: Gestell
- GF: Gasflaschen
- H: Hydraulikanlage
- HY: Hydraulikantriebsmotor für Erregerwelle
- K: Klappe (federunterstützt)
- KS: Klappstütze
- L: Lasche
- LI: Lichterblende klappbar
- LS: Laufsteg
- M: Mischschaufel
- MM: Benzin- / Dieselmotor
- Ö: Auslassöffnung (im Boden)
- PB: Prallblech
- R: Rollen
- RA: Förderband an Auslassöffnung Ö
- RÖ: Revisionsöffnung
- RR: Rohrrahmen (des Gestells G)
- RU: Rutsche
- S: Staplerlaschen
- SF: Sichtfenster (Zugöffnung)
- SZ: Sägezahn (Basis von M)
- T: Trommelkörper
- TI: Trommelisolierung
- T1: unterer Tragrahmen (unterster Stock)
- T2: oberer Tragrahmen (oberster Stock)
- T2V: Verriegelungsöffnung für Transportsicherung TS
- TS: Transportsicherungen für Rüttelsieb A
- X: zusätzlicher Brenner an Auslassöffnung Ö
- Z: Antriebsmittel (Zahnkranz)

## Patentansprüche

1. Vorrichtung zur thermischen Behandlung von Schüttgut, welche aufweist:
• ein mehrstöckiges Gestell (G) aus Rohrrahmen (RR), in welchem am unteren Tragrahmen (T1) im untersten Stock ein Trommelkörper (T) mit Mischschaufeln (M) gelagert und auf dem im Abstand darüber liegenden Tragrahmen (T2) im obersten Stock ein Rüttelsieb (A) angeordnet ist,
• eine Rutsche (RU) zur Zuführung des vorgesiebten Mischguts zu einer Trommelöffnung (E) des Trommelkörpers (T),
• einen außerhalb des Trommelkörpers (T) angeordneten Gasbrenner (B), dessen Flamme sich in das Innere des Trommelkörpers (T) erstreckt und
• ein im Innern des Trommelkörpers (T) oberhalb der Flamme angeordnetes Prallblech (PB).

2. Vorrichtung nach Anspruch 1, wobei der Trommelkörper (T) zylindrisch ist, sich auf dem unteren Tragrahmen (T1) im Wesentlichen horizontal erstreckt und im benachbarten Deckel (D) die Öffnung für die Flamme und im benachbarten Boden (BO) eine Öffnung (Ö) für den Auslass des Schüttguts aufweist.

3. Vorrichtung nach einem oder mehreren der Ansprüche 1 und Anspruch 2, wobei der Trommelkörper (T) freilaufend auf mehreren Rollen (R) gelagert ist und um seine zentrale Achse rotiert.

4. Vorrichtung nach einem oder mehreren der Ansprüche 1 bis 3, wobei in der Nähe des Deckels (D) am Mantel des Trommelkörpers (T) in Querrichtung umlaufend ein Antriebsmittel (Z) angeordnet ist.

5. Vorrichtung nach einem oder mehreren der Ansprüche 1 bis 4, wobei auf der Innenseite des Trommelkörpers (T) im Abstand zueinander Mischschaufeln (M) angeordnet sind.

6. Vorrichtung nach Anspruch 5, wobei die Mischschaufeln (M) im wesentlichen U-förmig ausgestaltet sind, dass an der Außenseite eines der Schenkel eine Lasche (L) angeordnet ist, welche an die jeweilige schraubenlinienförmig zueinander angeordnete Befestigungsvorrichtung an der Innenseite der Trommel (T) form- oder kraftschlüssig befestigt ist und dass die Basis der Mischschaufeln (M) auf einer Seite sägezahnförmig (SZ) ausgestaltet ist.

7. Vorrichtung nach einem oder mehreren der Ansprüche 1 bis 6, wobei der Gasbrenner (B) einen Flammenwächter aufweist, welcher die Ausbildung der Flamme überwacht und regelt.

8. Vorrichtung nach einem oder mehreren der Ansprüche 1 bis 7, wobei der Trommelkörper (M) aus einzelnen abgekanteten Teilabschnitten besteht, welche stoffschlüssig miteinander verbunden sind und dass die am Mantel umlaufende Abkantung (AK) in einem am Gestell (G) angeordneten Führungsmittel geführt werden.

9. Vorrichtung nach einem oder mehreren der Ansprüche 1 bis 8, wobei der untere Tragrahmen (T1) Räder aufweist.

10. Vorrichtung nach einem oder mehreren der Ansprüche 1 bis 9, wobei an der Trommelöffnung (E) des Trommelkörpers (T) eine federunterstützte Klappe (K) angebracht ist, auf welche das Schüttgut rutscht und welche sich bei einem voreingestellten Wert öffnet.

11. Vorrichtung nach einem oder mehreren der Ansprüche 1 bis 10, wobei der Trommelkörper (T) eine wärmeisolierende Beschichtung (TI) aufweist.

12. Vorrichtung nach einem oder mehreren der Ansprüche 1 bis 11, wobei die Vorrichtung auf einem Fahrzeuganhänger (FA) mit Anhängerkupplung (FK) und Fahrzeugdeichsel (FD) aufgebaut ist.

13. Verfahren zur thermischen Behandlung von Schüttgut für eine Vorrichtung nach Anspruch 1, bei dem:
a) das Schüttgut einem im obersten Stock eines mehrstöckigen Gestells (G) angeordneten Rüttelsieb (A) zugeführt wird,
b) das vorgesiebte Mischgut in einen Trommelkörper (T) eingefüllt wird,
c) in dem Trommelkörper (T) von der offenen Flamme eines Gasbrenners (B) im Inneren des Trommelkörpers (T) aufgeheizt wird und
d) das erhitzte Mischgut mittels schraubenlinienförmig zueinander angeordnete Mischschaufeln (M) vom Boden der Mischtrommel (T) nach oben und zur Auslassöffnung (Ö) gefördert wird.

14. Verfahren nach Anspruch 13, bei dem im Arbeitsschritt:
a1) mittels eines Förderbands (F) oder einer Förderschnecke (FS) das nach Korngröße gesiebte Grobgut abgefördert wird.

15. Verfahren nach Anspruch 13, bei dem ein Flammenwächter die Ausbildung der Flamme unterhalb des Prallblechs (PB) überwacht und regelt.
